# EUROPEAN PATENT SPECIFICATION

(11) **EP 1 847 285 B1**
(45) Date of publication and mention of the grant of the patent: **23.09.2009**
(21) Application number: 07114158.4
(22) Date of filing: 30.10.2003
(51) Int. Cl.: A61M 5/145

(54) **Syringe piston grip detection device for an injection system**
Vorrichtung zur Anzeige des Eingriffes des Kolbenstangenflansches einer Spritze in einem Injektionssystem
Dispositif d'affichage pour l'engagement du collet d'une seringue dans un système d'injection

(30) Priority: 04.02.2003 JP 2003027025
(43) Date of publication of application: 24.10.2007
(62) Divisional of application: 03292717.0
(73) Proprietor: Nemoto Kyorindo Co., Ltd., Tokyo (JP)
(72) Inventor: Sakakibara, Masahiro c/o Nemoto Kyorindo Co.,Ltd., Tokyo (JP); Kanetaka, Toshio c/o Nemoto Kyorindo Co.,Ltd., Tokyo (JP); Tanaka, Masafumi c/o Nemoto Kyorindo Co., Ltd., Tokyo (JP)
(74) Representative: Cabinet Plasseraud

(56) References cited:
- EP-A- 1 110 569
- US-A- 5 533 981
- US-A- 5 545 140
- US-A- 5 879 360

## Description

### BACKGROUND OF THE INVENTION

### 1. Field of the Invention:

The present invention relates to a liquid injection system for injecting a liquid from a liquid syringe into a subject with a liquid injector, and more particularly to a liquid injection system for individually holding and relatively moving a cylinder and a piston of a liquid syringe with a liquid injector.

### 2. Description of the Related Art:

Imaging diagnostic apparatus for capturing tomographic images as fluoroscopic images of subjects include CT (Computed Tomography) apparatus, MRI (Magnetic Resonance Imaging) apparatus, and PET (Positron Emission Tomography) apparatus. Medical apparatus for capturing blood vessel images as fluoroscopic images of subjects include CTA (CT Angiography) apparatus, MRA (MR Angiography) apparatus, and ultrasonograph.

When such an imaging diagnostic apparatus is used to capture a tomographic image of a subject, it is occasionally necessary to inject a liquid such as a contrast medium or a saline solution into the subject. There has been put to practical use a liquid injector for automatically injecting a liquid into a subject. Such a liquid injector has a drive motor and a slider mechanism, and employs a liquid syringe that is removably mounted.

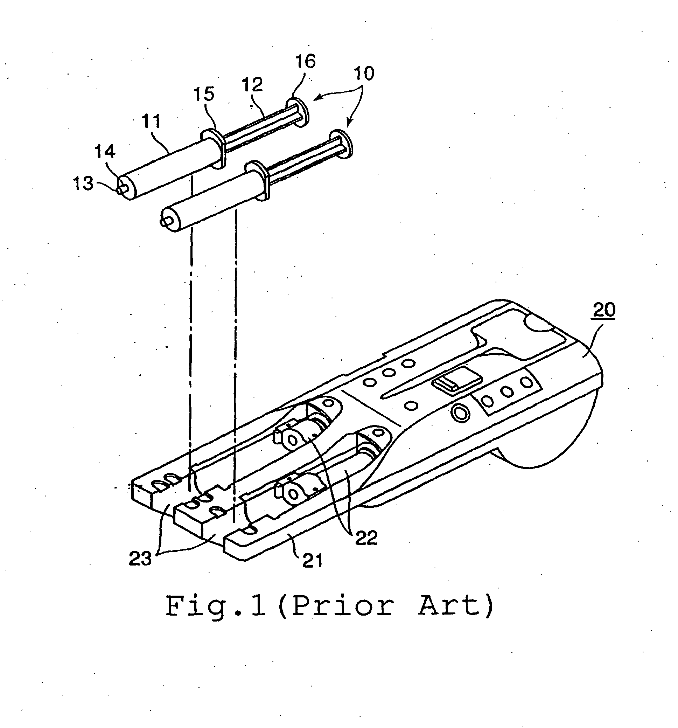

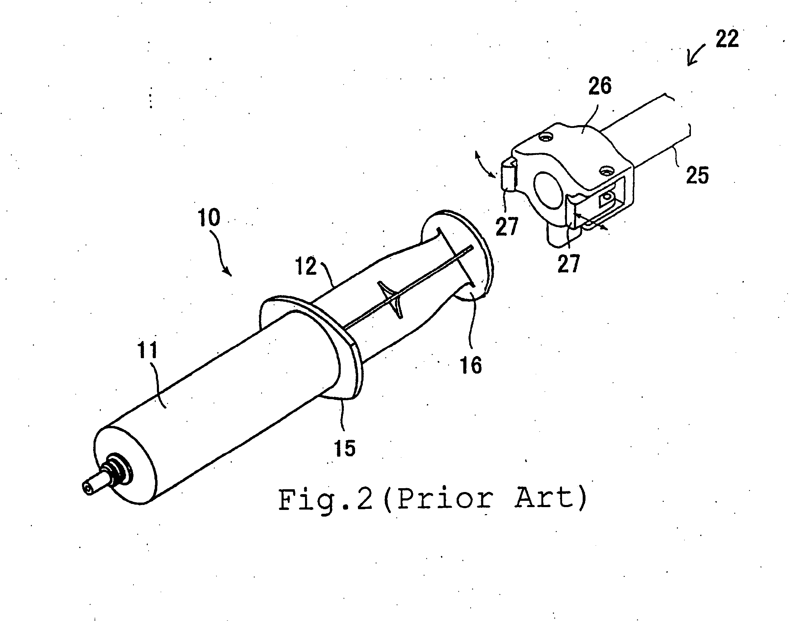

One conventional liquid injector will be described below with reference to Figs. 1 and 2 of the accompanying drawings. As shown in Fig. 1, conventional liquid injector 20 employs two liquid syringes 10 each comprising single cylinder 11 and single piston 12. Cylinder 11 has hole 13 defined therein which is open at an end face thereof.

The end face of cylinder 11 is closed with central hollow conduit 14 mounted thereon, and hole 13 communicates with the tip end of conduit 14. Piston 12 is slidably inserted in hole 13 in cylinder 11. Cylinder 11 and piston 12 have cylinder flange 15 and piston flange 16 respectively on and around their ends remote from conduit 14.

Liquid injector 20 has single injection head 21 serving as a cylinder gripping mechanism and two piston actuating mechanisms 22. Single injection head 21 has two recesses 23 defined therein for individually holding cylinders 11 of two liquid syringes 10. Two piston actuating mechanisms 22 are disposed respectively behind two recesses 23 for holding and sliding respective pistons 12 of liquid syringes 10.

More specifically, as shown in Fig. 2, each of piston actuating mechanisms 22 has slide rod 25 which is slidable back and forth and piston pusher 26 integrally formed with the front end of slide rod 25 for pushing piston 12 forwardly.

Each of piston actuating mechanisms 22 also has a pair of openable and closable engaging claws 27 mounted respectively on left and right sides of piston pusher 26. Engaging claws 27 are normally resiliently urged in a closing direction by respective resilient mechanisms such as helical springs. Engaging claws 27 have wedge-shaped distal ends for individually engaging left and right front edges of piston flange 16 when piston pusher 26 is pushed against piston 12 from the rear position.

Since conventional liquid injector 20 can inject two liquids from respective two liquid syringes 10 into a subject, it can inject a contrast medium, for example, into a subject who is to be imaged to capture a tomographic image with a CT scanner, and then inject a saline solution, for example, into the subject.

Conventional liquid injector 20 has its piston actuating mechanisms 22 positioned in a rear position in an initial state. After setting liquid syringes 10 in respective recesses 23, the operator makes an action to instruct liquid injector 20 to start injecting liquids into a subject. Piston pusher 26 of each piston actuating mechanism 22 is now moved forwardly.

When piston pusher 26 is pushed against piston 12, engaging claws 27 which have been resiliently brought into the closed position are pressed by piston flange 16 and progressively opened. As piston pusher 26 is moved further forwardly, engaging claws 27 engage the respective left and right front edges of piston flange 16, thus holding piston 12 with piston pusher 26. Since piston 12 is continuously pressed by piston actuating mechanism 22, the liquid is injected from liquid syringe 10 into the subject.

Because piston flange 16 is gripped by engaging claws 27 of piston pusher 26 as described above, when the operator makes a certain action if necessary, piston pusher 26 can be retracted to pull piston 12 out of cylinder 11.

According to IEC (international Electrotechnical Commission) regulations about the safety of medical electric apparatus, it is required that piston pusher 26 of liquid injector 20 hold piston 12 of liquid syringe 10.

Liquid injectors of the type described above have been devised and applied for patent by the applicant of the present application (see, for example, patent documents 1, 2 below).

Patent document 1: Japanese laid-open patent publication No. 2002-11096;

Patent document 2: Japanese laid-open patent publication No. 2002-102343.

With above liquid injector 20, when piston pusher 26 presses piston 12 of liquid syringe 10, piston flange 16 is automatically gripped by engaging claws . 27. However, the operator is required to visually confirm whether piston flange 16 has been gripped by engaging claws 27 or not.

If the operator falls to confirm the gripped engagement, then piston 12 may possibly be pushed by piston pusher 26 while piston flange 16 Is not being gripped by engaging claws 27. For example, If liquid syringe 10 Is not installed in proper position, then since piston 12 and piston pusher 26 are not in accurate central alignment with each other, the tip end faces of engaging claws 27 are held against the rear surface of piston flange 16, so that engaging claws 27 may not grip piston flange 16.

Furthermore, when piston 12 of liquid syringe 10 is pushed by piston pusher 26, piston flange 16 is gripped by engaging claws 27. Therefore, it is difficult for engaging claws 27 to grip piston flange 16 without having liquid syringe 10 discharges the liquid from cylinder 11.

US 5,533,981 discloses an infusion pump for dispensing fluid from a syringe according to the preamble of claim 1, having a barrel and a plunger slidably inserted into the barrel is disclosed. The infusion pump provides a housing having a bracket which supports the barrel. A syringe driver is movably attached to the housing such that the syringe driver abuts the plunger to slide the plunger into the barrel.

### SUMMARY OF THE INVENTION

It is therefore an object of the present invention as claimed to provide a liquid injection system which Is capable of detecting when a piston flange of a piston of a liquid syringe is gripped by engaging claws of a piston pusher of a liquid injector.

A liquid injection system according to the present invention as claimed has a liquid syringe and a liquid injector. The liquid syringe has a cylinder and a piston. The cylinder has a cylinder flange disposed on and around an end thereof, and the piston has a piston flange disposed on and around an end thereof. The piston is slidably inserted in the cylinder.

The liquid injector comprises a cylinder gripping mechanism, a piston pusher, a pair of engaging claws, and a gripping detecting means. The cylinder gripping mechanism grips the cylinder such that the cylinder has a longitudinal direction oriented forwardly and rearwardly. The piston pusher is supported slidably in forward and rearward directions for pushing the piston at least forwardly.

The engaging claws are laterally openably and closably mounted on the piston pusher for individually engaging left and right edges of a front face of the piston flange. The gripping detecting means detects when the piston flange is gripped by the engaging claws. After the piston flange of the piston is detected as being gripped by the engaging claws of the piston pusher, the liquid injector moves the cylinder flange and the piston flange relatively to each other to inject a liquid from the liquid syringe into a subject or draws a liquid from a liquid reservoir tank into the cylinder.

In the liquid injection system, the liquid injector which grips the cylinder with the engaging claws and presses the piston with the piston pusher can detect when the piston flange is gripped by the engaging claws. Therefore, the piston pusher is prevented from being actuated when the piston flange is not gripped by the engaging claws, for example.

The various means referred to in the present invention may be arranged to perform their stated functions, and may be implemented by dedicated pieces of hardware for performing the functions, data processing apparatus for performing the functions according to computer programs, functions achieved in data processing apparatus according to computer programs, or combinations thereof.

The various means referred to in the present invention are not required to be individually independent entities, and may be arranged such that a plurality of means may be constructed as a single apparatus, a certain means may be part of another means, or part of a certain means and part of another means overlap each other.

The terms used herein to describe directions, e.g., forwardly, rearwardly, left, right, vertically, etc., are merely employed for the purpose of clarifying the relative relationship of such directions, and should not be interpreted as being limitative with respect to directions at the time the apparatus according to the present invention are manufactured and used.

The above and other objects, features, and advantages of the present invention will become apparent from the following description with reference to the accompanying drawings which illustrate examples of the present invention.

### BRIEF DESCRIPTION OF THE DRAWINGS

Fig. 1 is a perspective view of a conventional liquid injector, showing the manner in which liquid syringes are set on the liquid injector;
Fig. 2 is a perspective view of a liquid syringe and a piston pusher of the conventional liquid injector;
Figs. 3a and 3b are fragmentary perspective views of a piston pusher of a liquid injector according to an embodiment of the present invention;
Figs. 4a and 4b are perspective views showing the manner in which a liquid syringe is set on an injection head of the liquid injector according to the embodiment of the present invention;
Fig. 5 is a perspective view of the liquid injector according to the embodiment of the present invention;
Fig. 6 is a perspective view of a CT scanner as an imaging diagnostic apparatus;
Fig. 7 is a block diagram of an electric circuit arrangement of the liquid injector according to the embodiment of the present invention;
Fig. 8 is a flowchart of a processing sequence of the liquid injector according to the embodiment of the present invention;
Fig. 9 is a sectional plan view of a piston pusher according to a first modification of the present invention;
Fig. 10 is a sectional plan view of an internal structure of a switch device as a gripping detecting means according to the first modification;
Fig. 11 is a sectional plan view of an internal structure of a switch device according to a second modification of the present invention;
Figs. 12a and 12b are sectional plan views of internal structures of switch devices according to third and fourth modifications, respectively, of the present invention;
Fig. 13 is a sectional plan view of a piston pusher according to a fifth modification of the present invention;
Fig. 14 is a sectional plan view of a piston pusher according to a sixth modification of the present invention;
Fig. 15 is a sectional plan view of a piston pusher according to a seventh modification of the present invention;
Fig. 16 is a sectional plan view of a piston pusher according to an eighth modification of the present invention;
Fig. 17 is a sectional plan view of a piston pusher according to a ninth modification of the present invention;
Fig. 18 is a sectional plan view of a piston pusher according to a tenth modification of the present invention;
Fig. 19 is a sectional plan view of a piston pusher according to an eleventh modification of the present invention;
Fig. 20 is a sectional plan view of a piston pusher according to a twelfth modification of the present invention;
Fig. 21 is a sectional plan view of a piston pusher according to a thirteenth modification of the present invention;
Fig. 22 is a sectional plan view of a piston pusher according to a fourteenth modification of the present invention; and
Fig. 23 is a sectional plan view of a piston pusher according to a fifteenth modification of the present invention.

### DETAILED DESCRIPTION OF THE PREFERRED EMBODIMENTS

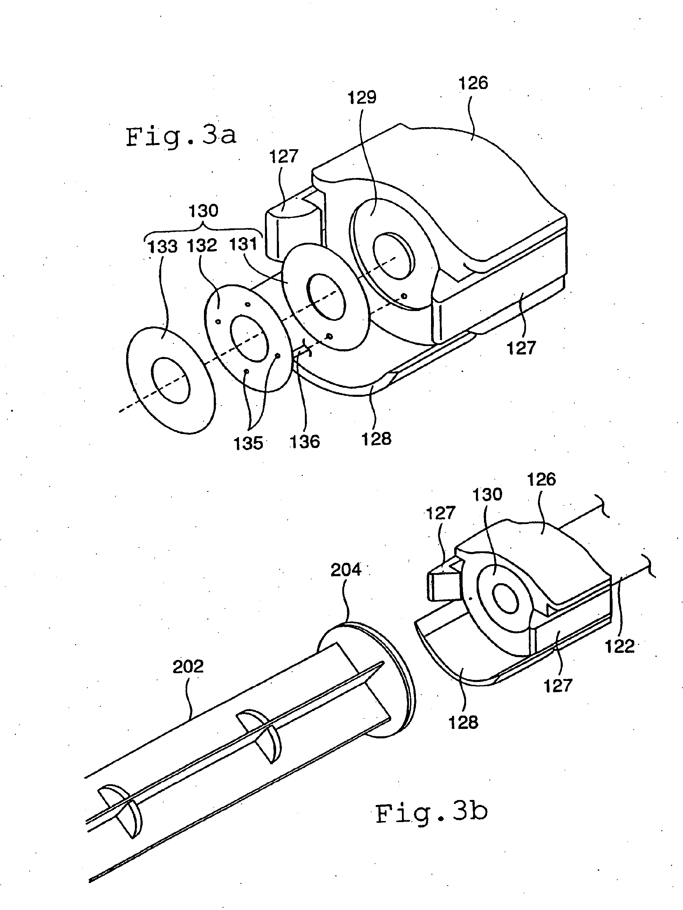

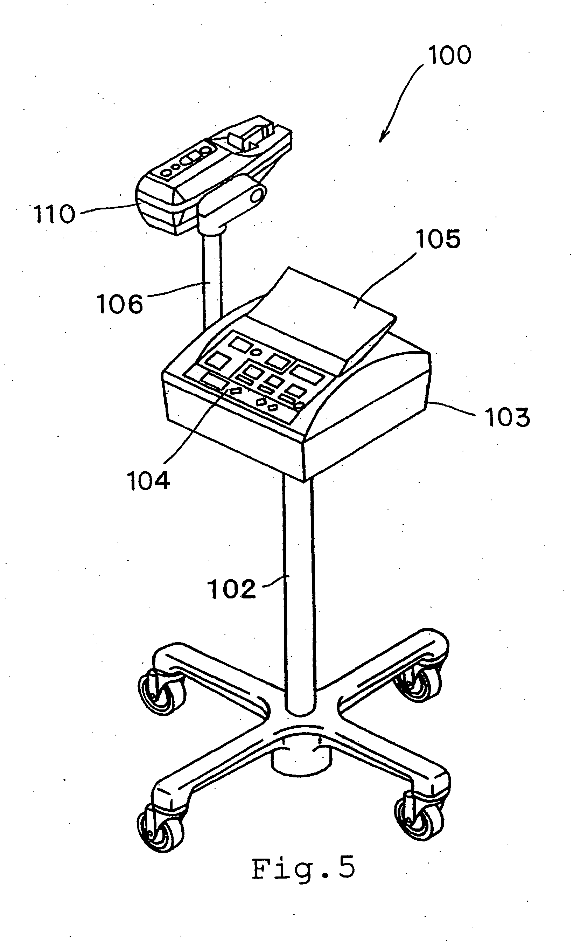

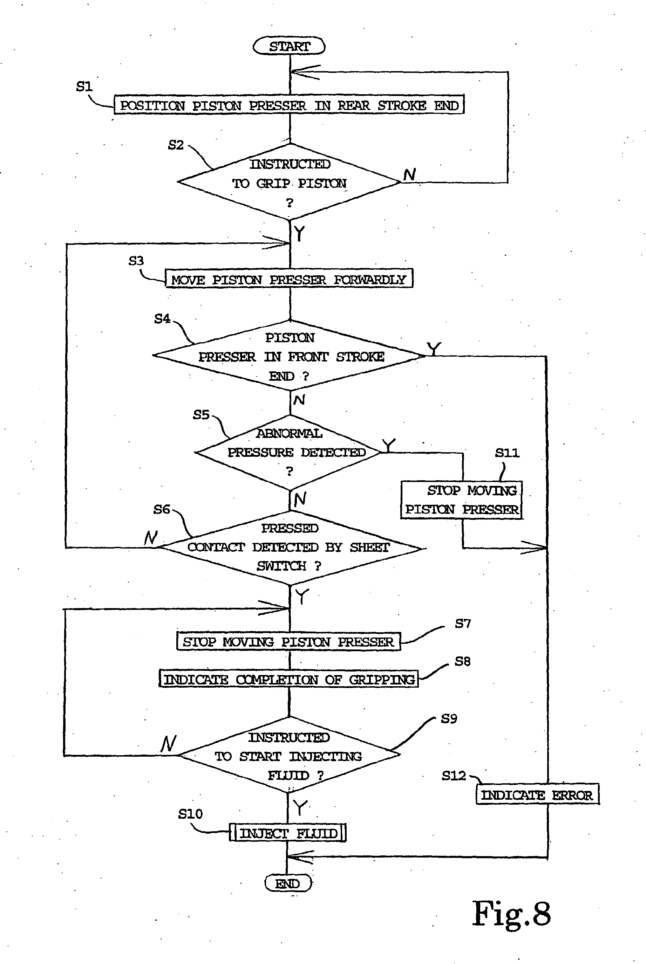

A liquid injector according to an embodiment of the present invention will be described below with reference to Figs. 3a, 3b through 8. As shown in Fig. 5, a liquid injector 100 according to an embodiment of the present invention has main body 103 mounted on the upper end of stand 102. Main body 103 supports thereon console panel 104 as an input operation device and liquid crystal display 105 as a data display device.

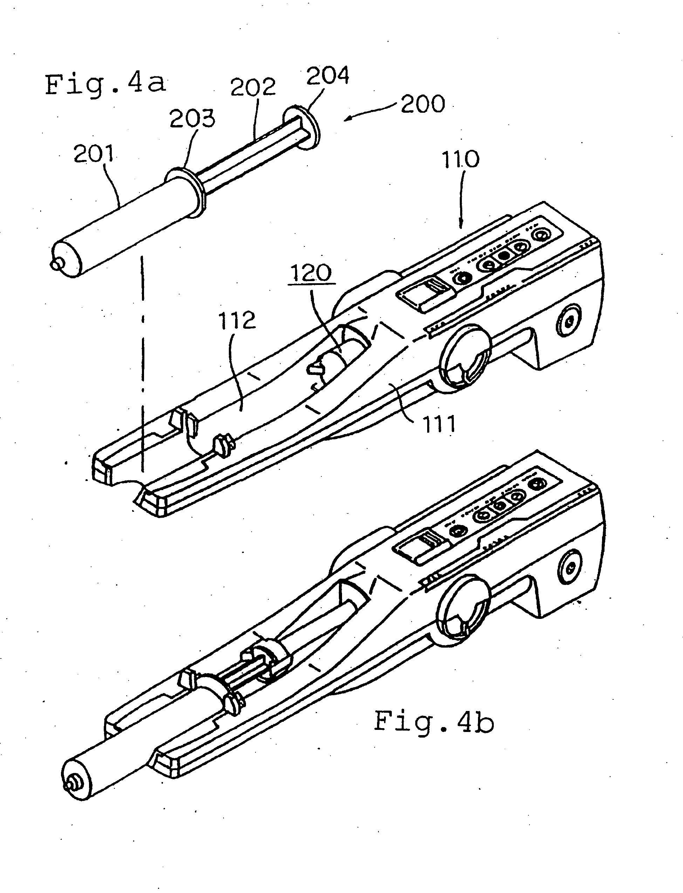

Arm 106 is vertically mounted on a side wall of main body 103, and injection head 110 as a cylinder gripping mechanism is mounted on the upper end of arm 106. As shown in Figs. 4a and 4b, injection head 110 has single recess 112 defined in an upper surface thereof. Cylinder 201 of liquid syringe 220 is removably held in recess 112.

Liquid syringe 200 comprises cylinder 201 and piston 202 slidably inserted in cylinder 201. Cylinder flange 203 is disposed on and around an end of cylinder 201, and piston flange 204 is disposed on and around an end of piston 202. Liquid injector 100 and liquid syringe 200 jointly make up a liquid injection system.

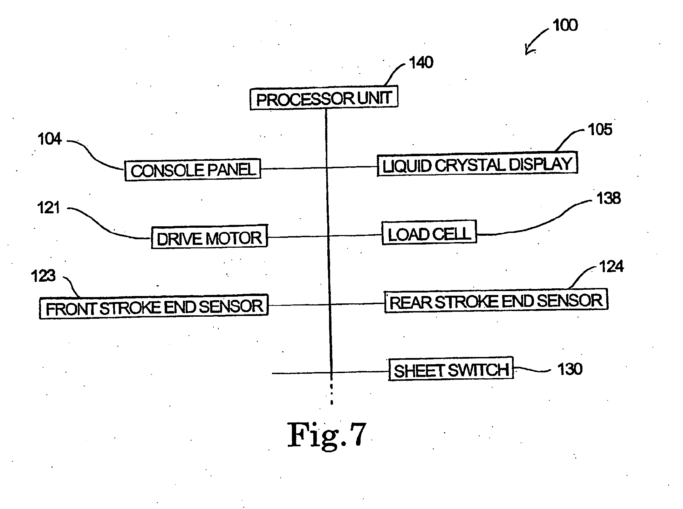

Piston actuating mechanism 120 is disposed behind recess 112 in injection head 110. Piston actuating mechanism 120 has drive motor 121 (see Fig. 7) such as an ultrasonic motor or the like for sliding slide rod 122 (see Fig. 3b) back and forth through a screw mechanism (not shown) or the like.

As shown in Fig. 7, piston actuating mechanism 120 also has front stroke end sensor 123 and rear stroke end sensor 124. Front stroke end sensor 123 detects when slide rod 122 reaches a front end of its stroke, and rear stroke end sensor 124 detects when slide rod 122 reaches a rear end of its stroke.

As shown in Figs. 3a and 3b, piston actuating mechanism 120 has piston pusher 126 integrally formed with the front end of slide rod 122, and a pair of openable and closable engaging claws 127 mounted respectively on left and right sides of piston pusher 126. Engaging claws 127 have wedge-shaped distal ends and are normally resiliently urged in a closing direction by respective resilient mechanisms such as helical springs.

Forwardly projecting guide 128 is integrally formed with a lower portion of piston pusher 126. Guide 128 has an upper surface which is of a conical shape slightly converging from its front end toward rear end. When guide 128 engages an outer circumferential surface of piston flange 204 as it is displaced relatively toward the front face of piston pusher 126, guide 128 guides piston flange 204 to an appropriate position with respect to piston pusher 126.

Piston pusher 126 has an annular recess 129 defined in the front face thereof. Sheet switch 130 serving as a gripping detecting means is mounted in recess 129. Sheet switch 130 comprises sheet board 131, switch sheet 132, and rubber cover 133. Four microswitches 135 are disposed on switch sheet 132.

Microswitches 135 are interconnected by a printed circuit board (not shown), and connected by wiring cable 136 to processor unit 140 (Fig. 7) through an A/D (Analog/Digital) converter (not shown).

In liquid injector 100 according to the present embodiment, sheet switch 130 detects when piston flange 204 is gripped by engaging claws 127 at the time microswitches 135 detect the rear face of piston 202 as it is pushed against the front face of piston pusher 126.

Therefore, the shape of piston flange 204, the shape of engaging claws 127, the shape of piston pusher 126, and the stroke of sheet switch 130 are adjusted such that sheet switch 130 detects piston 202 as it is pushed against piston pusher 126 when piston flange 204 is gripped by engaging claws 127.

Piston actuating mechanism 120 has load cell 138 in the junction between piston pusher 126 and slide rod 122 for detecting the pressure under which piston pusher 126 presses piston 202.

As shown in Fig. 7, liquid injector 100 has processor unit 140 connected to console panel 104, liquid crystal display 105, drive motor 121, front stroke end sensor 123, rear stroke end sensor 124, sheet switch 130, load cell 138, etc.

Processor unit 140 comprises a so-called one-chip microcomputer, and has a suitable computer program installed in the form of firmware. Processor unit 140 operates according to the installed computer program for controlling the various components connected thereto.

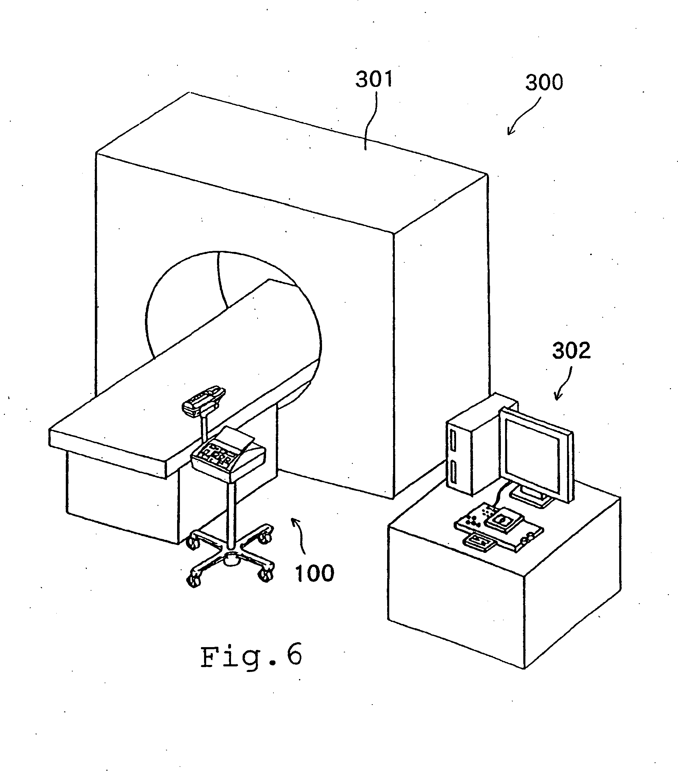

As shown in Fig. 6, liquid injector 100 is positioned near CT scanner 300 which serves as an imaging diagnostic apparatus. Liquid injector 100 injects a contrast medium as a liquid into a subject who is to be imaged by CT scanner 300. CT scanner 300 has imaging unit 301 and control unit 302 which is connected on-line to liquid injector 100.

For using liquid injector 100, as shown in Fig. 6, the operator (not shown) positions liquid injector 100 near CT scanner 300, and connects liquid syringe 200 to the subject who is positioned in imaging unit 301 with an extension tube (not shown).

The operator then places cylinder 201 of liquid syringe 200 in recess 112 of injection head 110, and makes an input action on console panel 104 to instruct liquid injector 100 to grip piston 202. When liquid injector 100 is in its initial state, piston pusher 126 of piston actuating mechanism 120 is positioned in a rear stroke end that is detected by rear stroke end sensor 124 in step S1. When liquid injector 100 is instructed to grip piston 202 in step S2, drive motor 121 is energized to move piston pusher 126 forwardly in step S3.

As piston pusher 126 is moved forwardly, it is pushed against piston 202 from behind piston 202, causing engaging claws 127 that are resiliently closed to be progressively opened by pressed contact with piston flange 204. Upon further forward movement of piston pusher 126, engaging claws 127 engage respective opposite edges of piston flange 204, so that piston pusher 126 grips piston 202.

When piston pusher 126 is pushed against piston 202 from behind piston 202, even if piston 202 is not properly located with respect to piston pusher 126, guide 128 engages the outer circumferential surface of piston flange 204 and forcibly guides piston flange 204 into an appropriate position where piston flange 204 can be gripped by engaging claws 127.

When piston flange 204 is thus gripped by engaging claws 127, the rear face of piston 202 is pressed against sheet switch 130, which detects the pressed contact by piston 202 in step S6. Then, drive motor 121 is de-energized to stop moving piston pusher 126 in step S7. Liquid injector 100 is now kept in a state where piston flange 204 of piston 202 of liquid syringe 200 is gripped by engaging claws 127 of piston pusher 126 of piston actuating mechanism 120.

If sheet switch 130 does not detect the pressed contact by piston 202, but front stroke end sensor 123 detects when piston pusher 126 has moved to its front stroke end in steps S6, S4, then it means that liquid syringe 200 is not set in recess 112 of injection head 110.

At this time, liquid injector 100 displays a guidance message such as "SYRINGE NOT DETECTED, CONFIRM IF SYRINGE IS NORMALLY SET OR NOT" on liquid crystal display 105 in step S12, indicating to the operator that liquid syringe 200 is not set in place.

If piston pusher 126 presses piston 202 without engaging claws 127 gripping piston flange 204 and, then the pressure applied by piston pusher 126 is detected by load cell 138 in step S5. In this case, liquid injector 100 de-energizes drive motor 121 in step S11, and displays a guidance message such as "PISTON NOT GRIPPED, REPEAT AGAIN FROM START" on liquid crystal display 105 in step S12, indicating to the operator that piston 202 has failed to be gripped.

If engaging claws 127 grip piston flange 204 and piston pusher 126 is stopped in steps S6, S7, then liquid injector 100 displays a guidance message such as "PISTON GRIPPING COMPLETED, INJECTION WILL BE STARTED WHEN xx KEY IS PRESSED" on liquid crystal display 105 in step S8, indicating to the operator that the gripping of piston 202 is completed.

If the operator makes an input action on console panel 104 to instruct liquid injector 100 to start injecting the liquid in step S9, then drive motor 121 is energized again to move piston pusher 126 forwardly, thus carrying out a process of injecting the liquid into the subject in the same manner as with conventional in step S10.

in the injecting process, the pressure under which the liquid is injected into the subject is calculated at all times from the pressure detected by the load cell 138. If the calculated injecting pressure falls out of a predetermined allowable range, then liquid injector 100 de-energizes drive motor 121 and displays an error guidance message on liquid crystal display 105.

When the operator makes an input action on console panel 104 to perform a liquid suction process while liquid syringe 200 is being connected to an external liquid reservoir tank (not shown), liquid injector 100 reverses drive motor 121 to retract piston pusher 126 for drawing a liquid from the external liquid reservoir tank into the liquid syringe 200.

With liquid injector 100 according to the present embodiment, when cylinder 201 of liquid syringe 200 is held by injection head 110 and piston 202 is pushed by piston pusher 126, sheet switch 130 detects the gripping of piston flange 204 with engaging claws 127. Therefore, piston pusher 126 is prevented from pushing piston 202 while piston flange 204 is not being gripped by engaging claws 127.

Particularly, when piston 202 is pushed by piston pusher 126, the pressure imposed on piston pusher 126 is monitored by load cell 138. If load cell 138 detects an abnormal pressure without sheet switch 130 detecting the pressed contact by piston flange 204, liquid injector 100 stops moving piston pusher 126. Accordingly, piston pusher 126 is automatically and reliably prevented from pushing piston 202 while piston flange 204 is not being gripped by engaging claws 127.

The gripping of piston flange 204 with engaging claws 127 is detected when sheet switch 130 disposed on the front face of piston pusher 126 is pushed by the rear face of piston 202. Thus, the gripping of piston flange 204 with engaging claws 127 is detected well by a simple structure.

With such a detecting structure, the detecting accuracy of sheet switch 130 is lowered if the rear face of piston 202 is brought into proper abutment against the front face of piston pusher 126. Liquid injector 100 according to the present embodiment keeps the detecting accuracy of sheet switch 130 at a high level because guide 128 of piston pusher 126 guides piston flange 204 into an appropriate position for reliable contact with piston pusher 126.

With the liquid injection system according to the present invention, the mechanism for detecting when piston flange 204 is gripped by engaging claws 127 is contained in liquid injector 100 themselves. Therefore, liquid syringe 200 is not required to incorporate a special design for detecting the gripping of piston flange 204, but may be in the form of a commercially available product.

The present invention is not limited to the above embodiment, but various changes and modifications may be made therein without departing from the scope thereof. For example, while illustrated liquid injector 100 allows single liquid syringe 200 to be set on injection head 110, the present invention is also applicable to a liquid injector (not shown) capable of holding a plurality of liquid syringes 200 on the injection head.

In the illustrated embodiment, sheet switch 130 disposed on the front face of piston pusher 126 detects whether piston 202 is pushed by piston pusher 126 or not based on a binary signal. However, the pressure under which piston 202 is pushed by piston pusher 126 may be detected in an analog fashion by a sheet sensor (not shown).

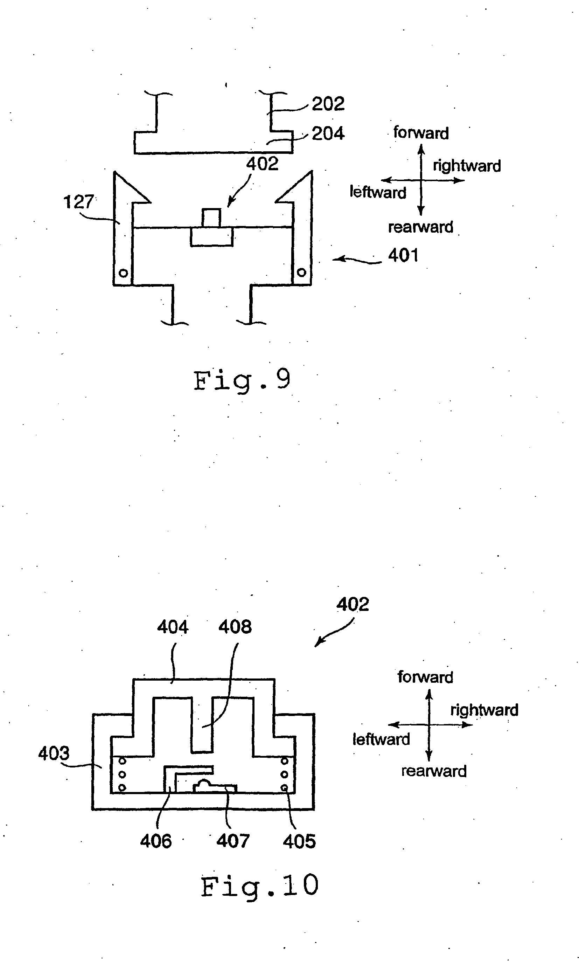

In illustrated embodiment, sheet switch 130 which is dedicated as the gripping detecting means is disposed on the front face of piston pusher 126. However, as shown in Fig. 9, commercially available switch device 402 or sensor device (not shown) may be mounted as a gripping detecting means on the front face of piston pusher 401.

As shown in Fig. 10, switch device 402 comprises switch casing 403 as a member support means, displacement detecting member 404, helical spring 405 as a member biasing means, and a pair of electrode terminals 406, 407 as a retraction detecting means. Displacement detecting member 404 against which the rear face of piston 202 is supported by switch casing 404 for forward and rearward movement, and is normally biased to move forwardly by helical spring 405.

Electrode terminals 406, 407 are spaced from, but confront, each other in the forward and rearward directions. Displacement detecting member 404 has arm 408 projecting from a rear surface thereof in facing relation to front electrode terminal 406. When displacement detecting member 404 is retracted to a predetermined position, arm 408 pushes electrode terminal 406 into contact with electrode terminal 407, whereupon electrode terminals 406, 407 are short-circuited. In a liquid injector (not shown) which incorporates above switch device 204 mounted on piston pusher 401, the gripping of piston flange 204 with engaging claws 127 can be detected by a simple structure that comprises commercially available components.

In the illustrated embodiment, guide 128 projects forwardly from the lower portion of piston pusher 126 for guiding piston flange 204 to the appropriate position. However, a convex and a concave may be provided as a guide mechanism on the rear face of the piston and the front face of the piston pusher.

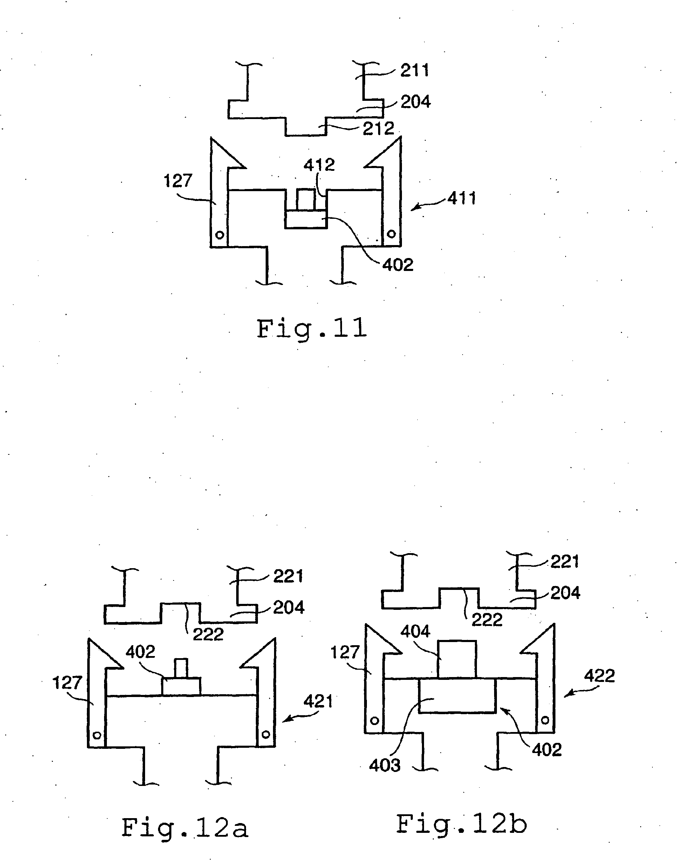

Such a convex/concave structure as a guide mechanism may be combined with switch device 402 shown in Fig. 10. Specifically, as shown in Fig. 11, convex 212 is formed centrally on the rear face of piston 211, and concave 412 is defined centrally in the front face of piston pusher 411, with switch device 402 disposed in concave 412.

The convex/concave structure can guide piston 211 to an appropriate position with respect to piston pusher 411, so that switch device 402 can reliably be operated using the convex/concave structure as the guide mechanism. For guiding piston 211 to an appropriate position with respect to piston pusher 411, each of convex 212 and concave 412 should preferably be of a conically tapered shape.

As shown in Fig. 12(a), a concave 222 may be defined centrally in the rear face of piston 221, and switch device 402 may be disposed as a convex centrally on the front face of piston pusher 421. Alternatively, as shown in Fig. 12(b), switch device 402 may be mounted centrally on the front face of piston pusher 422 with displacement detecting member 404 provided as a convex.

In switch device 402 shown as the gripping detecting means in Fig. 10, electrode terminals 406, 407 that are spaced from each other serve as the retraction detecting means and are short-circuited when displacement detecting member 404 is retracted. The arrangement shown in Fig. 10 may be modified in various ways.

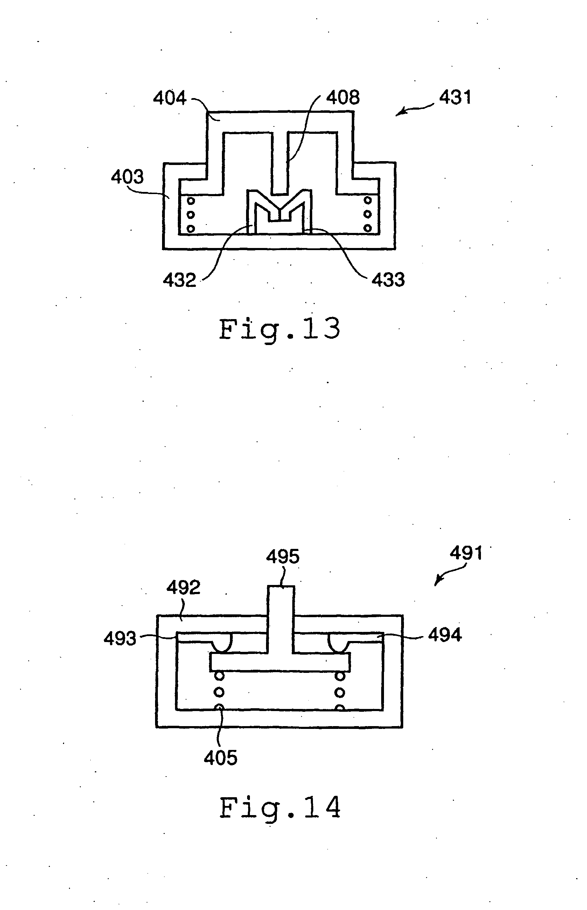

For example, Fig. 13 shows switch device 431 having a pair of electrode terminals 432, 433 mounted as a retraction detecting means on switch casing 403 and resiliently held in a short-circuited position. Electrode terminals 432, 433 are separable from each other when arm 408 of displacement detecting member 404 is forced in between electrode terminals 432, 433 as displacement detecting member 404 is retracted.

Fig. 14 shows switch device 491 having a pair of electrode terminals 493, 494 mounted as a retraction detecting means on a rear face of a front panel of switch casing 492, and electrically conductive displacement detecting member 495 short-circuited to electrode terminals 493, 494. When displacement detecting member 495 is retracted, it is spaced from electrode terminals 493, 494.

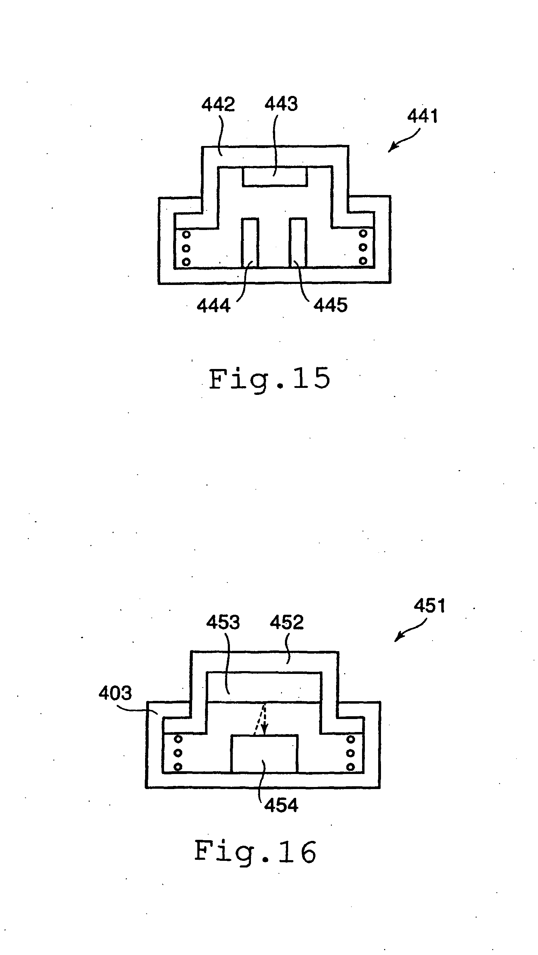

Fig. 15 shows switch device 441 having electrically conductive member 443 mounted on a rear face of displacement detecting member 442, and a pair of electrode terminals 444, 445 that are horizontally or vertically spaced from each other. Electrode terminals 444, 445 are short-circuited to each other by electrically conductive member 443 when displacement detecting member 442 is retracted.

Fig. 16 shows switch device 451 having reflecting plate 453 mounted on a rear face of displacement detecting member 452, and optical range finder 454 mounted as a wave range finder device on switch casing 403. Optical range finder 454 optically measures the distance up to reflecting plate 453 with a wave such as visible light or infrared rays.

If the rear face of displacement detecting member 452 functions as a good reflecting surface, then reflecting plate 453 may be dispensed with. Furthermore, optical range finder 454 may be replaced with an ultrasonic range finder as a wave range finder device, and the distance up to displacement detecting member 452 may be measured by the ultrasonic range finder using an ultrasonic energy as a wave.

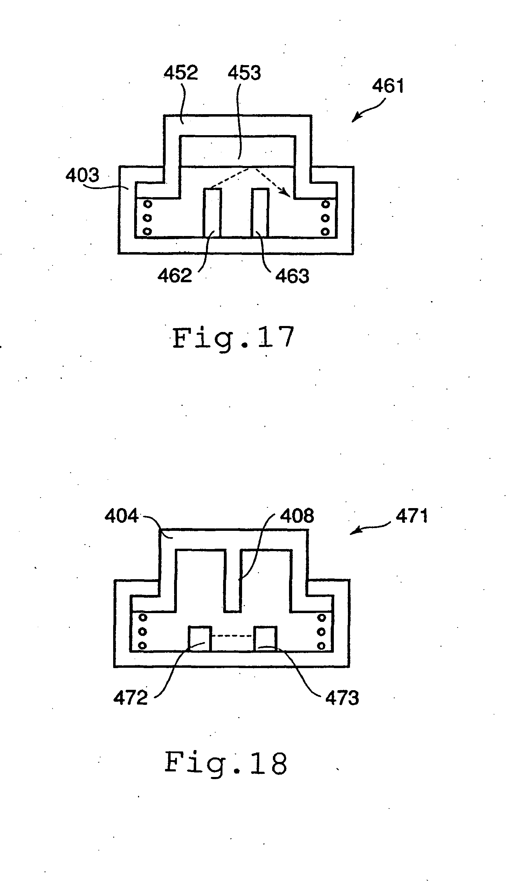

Fig. 17 shows switch device 461 having light-emitting element 462 for emitting a collimated light beam such as a laser beam at a predetermined angle, and light-detecting element 463 for detecting a light beam applied at a predetermined angle, light-emitting element 462 and light-detecting element 463 being mounted on switch casing 403. When displacement detecting member 452 is retracted to a predetermined position, a light beam emitted from light-emitting element 462 and reflected by reflecting plate 453 is applied to light-detecting element 463.

Fig. 18 shows switch device 471 having light-emitting element 472 and light-detecting element 473 which are mounted on switch casing 403 in confronting relation to each other. A light beam emitted from light-emitting element 472 and applied to light-detecting element 473 is interrupted by arm 408 of displacement detecting member 404 when displacement detecting member 404 is retracted.

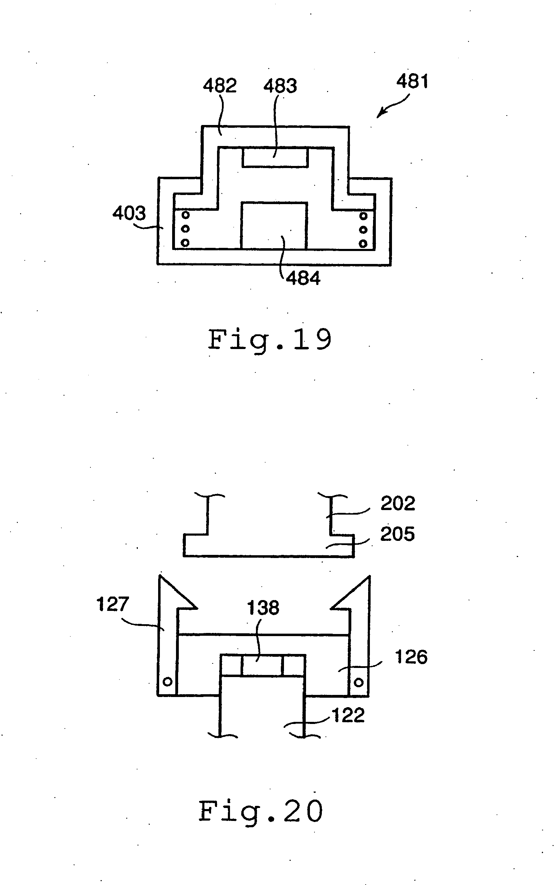

Fig. 19 shows switch device 481 having magnet 483 mounted on a rear face of displacement detecting member 482, and Hall device 484 mounted on switch casing 403 for detecting magnet 483. In each of the above switch devices, the components positioned on the displacement detecting member and the components positioned on the switch casing may be switched around, e.g., convex 208 may be disposed on switch casing 403, and electrode terminals 406, 407 may be mounted on displacement detecting member 404.

Since each of the above switch devices is capable of detecting when the displacement detecting member is retracted to a predetermined position, it can detect when piston 202 of existing liquid syringe 200 which are not specially designed is pressed. The internal structure of each of the above switch devices may be incorporated in piston 202 of liquid syringe 200 and piston pusher 126.

For example, arm 408 of switch device 402 shown in Fig. 10 may be disposed on the rear face of piston 202, and electrode terminals 406, 407 may be disposed on the front face of piston pusher 126. Electrode terminals 432, 433 shown in Fig. 13 may be disposed on the front face of piston pusher 126.

Electrically conductive member 443 shown in Fig. 15 may be disposed on the rear face of piston 202, and electrode terminals 444, 445 may be disposed on the front face of piston pusher 126. Reflecting plate 453 shown in Fig. 16 may be disposed on the rear face of piston 202, and optical range finder 454 or light-emitting and -detecting elements 462, 463 shown in Fig. 17 may be disposed on the front face of piston pusher 126.

Arm 408 shown in Fig. 18 may be disposed on the rear face of piston 202, and light-emitting and -detecting elements 472, 473 may be disposed on the front face of piston pusher 126. Magnet 483 shown in Fig. 19 may be disposed on the rear face of piston 202, and Hall device 484 may be disposed on the front face of piston pusher 126.

If electrode terminals 444, 445 exposed on the front face of piston pusher 126 poses a problem, then, as shown in Fig. 11, electrode terminals 444, 445 may be disposed in concave 412 in piston pusher 126.

In the above embodiment, sheet switch 130 which is a dedicated piece of hardware as the gripping detecting means is disposed on the front face of piston pusher 126. However, the gripping detecting means may be implemented by a change of software without the need for a change in existing hardware.

For example, as shown in Fig. 20, liquid injector 100 has load cell 138 disposed in the junction between piston pusher 126 and slide rod 122 for detecting an abnormal pressure of the liquid being injected. Since the pressure detected by load cell 138 changes even when engaging claws 127 abut against and then grip piston flange 204 upon forward movement of piston pusher 126, it is possible to detect when engaging claws 127 grip piston flange 204 from the change in the pressure.

For better detecting accuracy, it is preferable to store data representative of a pattern of pressure changes which occur after engaging claws 127 abut against piston flange 204 until engaging claws 127 grip piston flange 204, and to carry out a pattern recognition process on changes in the pressure detected by load cell 138 based on the stored data for thereby detecting when engaging claws 127 grip piston flange 204.

Alternatively, a pattern of pressure changes detected by load cell 138 may be displayed as a chronological graph in real-time on liquid crystal display 105 for enabling the operator to visually confirm the gripping of piston flange 204 with engaging claws 127.

While the liquid is being injected into the subject, pressure changes may be displayed to enable the operator to confirm an abnormal pressure buildup. A small-size display panel (not shown) dedicated to the display of such pressure changes may be positioned next to injection head 110 for better operation of the liquid injection system.

In the illustrated embodiment, in order to detect the gripping of piston flange 204 with engaging claws 127, sheet switch 130 serving as the gripping detecting means detects when the rear face of piston 22 is pushed against the front face of piston pusher 126.

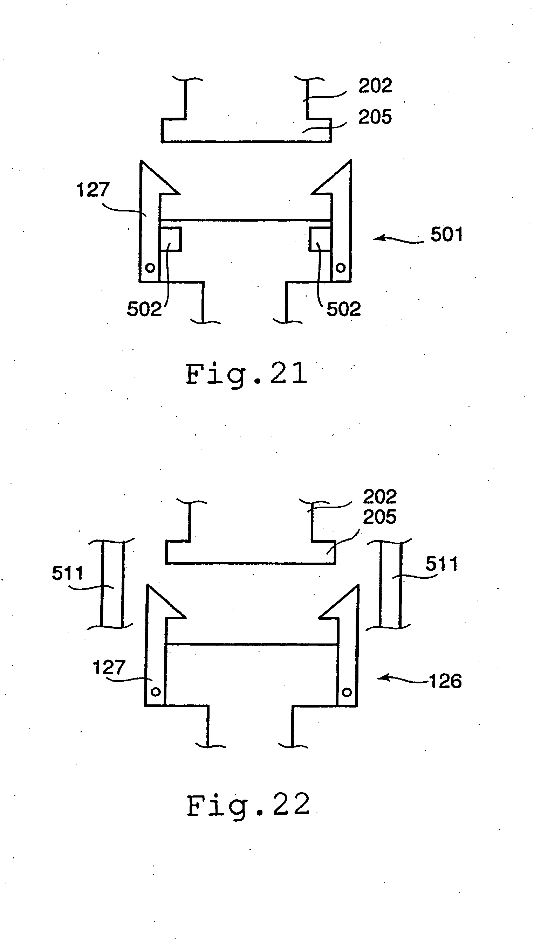

However, as shown in Fig. 21, piston pusher 501 may have a pair of switch devices 502 disposed as a gripping detecting means on respective outer sides. Switch devices 502 detect when engaging claws 127 are opened from the initial closed position into opposite sides and then closed, thus detecting the gripping of piston flange 204 with engaging claws 127.

For better detecting accuracy, if switch devices 502 detects only the opening and closing of engaging claws 127 with a binary signal, then it is preferable to store data representing a period of time that elapses from the opening of engaging claws 127 to the closing of engaging claws 127, and to compare the stored data with a detecting time of switch devices 502.

Each of switch devices 502 may be of the same structure as sheet switch 130 or switch device 402. For example, each of switch devices 502 may be replaced with an analog sensor device (not shown).

Such an analog sensor device can detect the opening and closing operation of engaging claws 127 in an analog fashion. Therefore, data representing a pattern of changes in the opening and closing movement of engaging claws 127 may be stored, and the action of engaging claws 127 as they are opened from the initial closed position into opposite sides and then closed may reliably be detected according to a pattern recognition process based on the stored data.

Further alternatively, as shown in Fig. 22, switch devices 511, each comprising a number of sheet-like touch switches mounted in succession on a tape, may be disposed as a gripping detecting means on both sides of piston pusher 126 for detecting when engaging claws 127 are opened and closed.

Switch devices 511 are capable of detecting when they are pressed at any position thereon in the longitudinal direction thereof. Therefore, switch devices 511 can detect when piston pusher 126 grips piston 202 in different positions along the longitudinal direction of switch devices 511. The arrangement shown in Fig. 22 is relatively simple and can be manufactured with ease because no gripping detecting means needs to be added to piston pusher 126 which is a movable mechanism. "Tape switch" (registered trade mark) manufactured by Tokyo Sensor CO., LTD. may be used as such switch devices 511, for example.

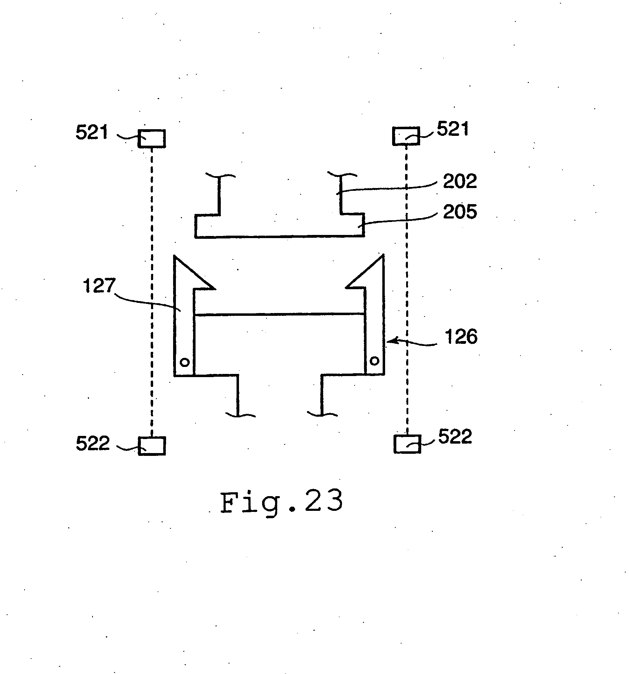

As shown in Fig. 23, a pair of light-emitting elements 521 may be disposed in respective positions where they emit light beams along paths that can be interrupted by engaging claws 127 as they are opened, and a pair of light-detecting elements 522 may be disposed in respective positions where they detect light beams emitted from respective light-emitting elements 521 along the paths that are not interrupted by engaging claws 127. Light-emitting elements 521 and light-detecting elements 522 are effective to detect engaging claws 127 as they are opened and closed.

The arrangement shown in Fig. 23 is also capable of detecting when piston pusher 126 grips piston 202 in different positions along the longitudinal direction, and is relatively simple and can be manufactured with ease because no gripping detecting means need to be added to piston pusher 126 which is a movable mechanism.

The various gripping detecting means described above may be combined together to increase the accuracy with which to detect when piston flange 204 is gripped by engaging claws 127. For example, pressure changes may be detected by load cell 128, and the opening and closing of engaging claws 127 may be detected by switch devices 502. If the detecting timings of load cell 128 and switch devices 502 match each other, then it is possible to detect when piston flange 204 is gripped by engaging claws 127 with increased accuracy.

While preferred embodiments of the present invention have been described using specific terms, such description is for illustrative purposes only, and it is to be understood that changes and variations may be made without departing from the spirit or scope of the following claims.

## Claims

1. A liquid injection system comprising:
a liquid syringe (200) having a cylinder (201) with a cylinder flange (203) disposed on and around an end thereof and a piston (202) slidably inserted In said cylinder (201) and having a piston flange (204) disposed on and around an end thereof; and
a liquid Injector (100) for holding and moving at least said cylinder flange (203) and said piston flange (204) relatively to each other;
said liquid injector (100) comprising a cylinder gripping mechanism (110) for gripping said cylinder (201) such that said cylinder (201) has a longitudinal direction oriented forwardly and rearwardly, a piston pusher (126) supported slidably in forward and rearward directions for pushing said piston (202) at least forwardly, a pair of engaging claws (127) laterally openably and closably mounted on said piston pusher (126) for individually engaging left and right edges of a front face of said piston flange (204), and gripping detecting means (130) for detecting when said piston flange (204) is gripped by said engaging claws (127),
**characterized in that** said gripping detecting means comprises means for detecting when said engaging claws are opened from an initial closed position into opposite sides and then closed.

2. A liquid Injection system according to claim 1, wherein said gripping detecting means comprises means (502) positioned on an outer surface of said piston pusher (501) for detecting when said piston flange (204) Is pressed by an Inner surface of each of said engaging claws (127).

3. A liquid injection system according to claim 1, wherein said gripping detecting means comprises means positioned outside of said piston pusher (126) for detecting when said piston flange (204) Is pressed by each of said engaging claws (127) which are opened.

4. A liquid Injection system according to claim 3, wherein said gripping detecting means (511) comprises a number of sheet-like touch switches mounted in succession on a tape.

5. A liquid injection system according to claim 1, wherein said gripping detecting means comprises:
a pair of light-emitting elements (521) for emitting respective light beams along paths Interruptable by said engaging claws (127) which are opened; and
a pair of light-detecting elements (522) for detecting light beams emitted respectively from said light-emitting elements along paths which are not interrupted by said engaging claws (127).

6. A liquid injector (100) for injecting a liquid from a liquid syringe (200) having a cylinder (201) with a cylinder flange (203) disposed on and around an end thereof and a piston (202) slidably inserted In said cylinder (201) and having a piston flange (204) disposed on and around an end thereof, said liquid injector (100) being arranged to hold and move at least said cylinder flange (203) and said piston flange (204) relatively to each other, said liquid injector (100) comprising:
a cylinder gripping mechanism (110) for gripping said cylinder (201) such that said cylinder (201) has a longitudinal direction oriented forwardly and rearwardly;
a piston pusher (126) supported slidably in forward and rearward directions for pushing said piston (202) at least forwardly:
a pair of engaging claws (127) laterally openably and closably mounted on said piston pusher (126) for individual engaging left and right edges of a front face of said piston flange (204); and
gripping detecting means for detecting when said piston flange (204) is gripped by said engaging claws (127),
**characterized in that** said gripping detecting means comprises means for detecting when said engaging claws (127) are opened from an initial closed position into opposite sides and then closed.

## Patentansprüche

1. Flüssigkeitsinjektionssystem, umfassen:
eine Flüssigkeitsspritze (200), die einen Zylinder (201) mit einem Zylinderflansch (203), der auf einem und um ein Ende desselben angeordnet ist, und einen Kolben (202) aufweist, der in dem Zylinder (201) verschiebbar eingesetzt ist und einen Kolbenflansch (204) aufweist, der auf einem und um ein Ende desselben angeordnet ist; und
einen Flüssigkeitsinjektor (100), um mindestens den Zylinderflansch (203) und den Kolbenflansch (204) relativ zueinander zu halten und zu bewegen;
wobei der Flüssigkeitsinjektor (100) umfasst: einen Zylindergreifmechanismus (110), um den Zylinder (201) so zu greifen, dass der Zylinder (201) eine Längsrichtung aufweist, die nach vorn und nach hinten ausgerichtet ist, eine Kolbenschiebevorrichtung (126), die in Vorwärts-und Rückwärtsrichtung verschiebbar getragen wird, um den Kolben (202) zumindest nach vom zu schieben, ein Paar von Eingriffsklauen (127), die sich seitlich öffnen und schließen lassen, die auf der Kolbenschiebevorrichtung (126) angebracht sind, um einzeln einen linken und rechten Rand einer Vorderseite des Kolbenflanschs (204) in Eingriff zu nehmen, und eine Greifdetektionseinrichtung (130), um zu detektieren, wenn der Kolbenflansch (204) durch die Eingriffsklauen (127) festgehalten wird,
**dadurch gekennzeichnet, dass** die Greifdetektionseinrichtung eine Einrichtung umfasst, um zu detektieren, wenn die Eingriftsklauen von einer geschlossenen Anfangsstellung nach entgegengesetzten Seiten geöffnet und dann geschlossen sind.

2. Flüssigkeitsinjektionssystem nach Anspruch 1, bei dem die Greifdetektionseinrichtung eine Einrichtung (502) umfasst, die auf einer Außenfläche der Kolhenschiebevorrichtung (501) positioniert ist, um zu detektieren, wenn der Kolbenflansch (204) durch eine Innenfläche von jeder der Eingriffsklauen (127) gepresst wird.

3. Flüssigkeitsinjektionssysten nach Anspruch 1, bei dem die Greifdetektionseinrichtung eine Einrichtung umfasst, die außerhalb der Kolhenschiebevorrichtung (126) positioniert ist, um zu detektieren, wenn der Kolbenflansch (204) durch jede der Eingriffsklauen (127) gepresst wird, die geöffnet sind.

4. Flüssigkeitsinjektionssystem nach Anspruch 3, bei dem die Greifdetektionseinrichtung (511) eine Anzahl von plattenartigen Berührungsschaltern umfasst, die auf einem Band hintereinander angebracht sind.

5. Flüssigkeitsinjektionssystem nach Anspruch 1, bei dem die Greifdetektionseinrichtung umfasst:
ein Paar von lichtemittierenden Elementen (521), um jeweilige Lichtstrahlen entlang Wegen zu emittieren, die sich durch die Eingriffsklauen (127) unterbrechen lassen, die geöffnet sind; und
ein Paar von Lichtdetektionselementen (522), um Lichtstrahlen zu detektieren, die jeweils von den lichtemittierenden Elementen entlang Wegen emittiert werden, die nicht durch die Eingriffsklauen (127) unterbrochen sind.

6. Flüssigkeitsinjektor (100) zum Injizieren einer Flüssigkeit aus einer Flussigkeitsspritze (200), die einen Zylinder (201) mit einen, Zylinderflansch (203), der auf einem und um ein Ende desselben angeordnet ist, und einen Kolben (202) aufweist, der in dem Zylinder (201) verschiebbar eingesetzt ist und einen Kolbenflansch (204) aufweist, der auf einem und um ein Ende desselben angeordnet ist, wobei der Flussigkeitsinjektor (100) angeordnet ist, um mindestens den Zylinderflansch (203) und den Kolbenflansch (204) relativ zueinander zu halten und zu bewegen, wobei der Flussigkeitsinjektor (100) umfasst:
einen Zylindergreifmechanismus (110), um den Zylinder (201) so zu greifen, dass der Zylinder (201) eine Längsrichtung aufweist, die nach vom und nach hinten ausgerichtet ist;
eine Kolbenschiebevorrichtung (126), die in Vorwärts- und Rückwärtsrichtung verschiebbar getragen wird, um den Kolben (202) zumindest nach vorn zu schieben;
ein Paar von Eingriffsklauen (127), die sich seitlich öffnen und schließen lassen, die auf der Kolbenschiebevorrichhmg (126) angebracht sind, um einzeln einen linken und rechten Rand einer Vorderseite des Kolbenflanschs (204) in Eingriff zu nehmen; und
eine Greifdetektionseinrichtung, um zu detektieren, wenn der Kolbenflansch (204) durch die Eingriffsklauen (127) festgehalten wird,
**dadurch gekennzeichnet, dass** die Greifdetektionseinrichtung eine Einrichtung umfasst, um zu detektieren, wenn die Eingriffsklauen (127) von einer geschlossenen Anfangsstellung nach entgegengesetzten Seiten geöffnet und dann geschlossen sind.

## Revendications

1. Système d'injection de liquide comprenant :
une seringue de liquide (200) ayant un cylindre (201), avec une bride de cylindre (203) disposée sur et autour d'une extrémité de celui-ci et un piston (202) inséré en coulissement dans ledit cylindre (201) et ayant une bride de piston (204) disposée sur et autour d'une extrémité de celui-ci, et
un injecteur de liquide (100) pour détenir et déplacer au moins ladite bride de cylindre (203) et ladite bride de piston (204) l'une par rapport à l'autre ;
ledit injecteur de liquide (100) comprenant un mécanisme de saisie du cylindre (1 10)
pour saisir ledit cylindre (201), de sorte que ledit cylindre (201) ait une direction longitudinale orientée vers l'avant et vers l'arrière, un système de poussée de piston (126) supporté en coulissement dans les directions vers l'avant et vers l'arrière pour pousser ledit piston (202) au moins vers l'avant, une paire de griffes de prise (127) montées de façon à pouvoir s'ouvrir et se fermer latéralement, sur ledit système de poussée de piston (126), afin de mettre en prise individuellement les bords gauche et
droit d'une face avant de ladite bride de piston (204) et des moyens de détection de prise (130) pour détecter quand ladite bride de piston (204) est saisie par lesdites griffes de prise (127),
**caractérisé en ce que** ledit système de détection de prise comprend des moyens pour détecter quand lesdites griffes de prise sont ouvertes, à partir d'une position fermée initiale sur des côtés opposés et ensuite fermées.

2. Système d'injection de liquide selon la revendication 1, dans lequel lesdits moyens de détection de prise comprennent des moyens (502) situés sur une surface extérieure dudit système de poussée du piston (501), afin de détecter quand ladite bride de piston (204) est comprimés par une surface interne de chacune desdites griffes de prises (127).

3. Système d'injection de liquide selon la revendication 1, dans lequel lesdits moyens de détection de prise comprennent des moyens positionnés à l'extérieur dudit système de poussée de piston (126), afin de détecter quand ladite bride de piston (204) est comprimée par chacune desdites griffes de prise (127) qui sont ouvertes.

4. Système d'injection de liquide selon la revendication 3, dans lequel lesdits moyens de défection de prise (511) comprennent un certain nombre de commutateurs tactiles semblables à des feuillés montés en succession sur une bande.

5. Système d'injection de liquide selon la revendication 1, dans lequel lesdits moyens de détection de prise comprennent :
une paire d'éléments électroluminescents (521) pour émettre des faisceaux lumineux respectifs, le long de chemins pouvant être interrompus par lesdites griffes de prise (127) qui sont ouvertes ; et
une paire d'éléments détecteurs de lumière (522) pour détecter les faisceaux lumineux émis respectivement par lesdits éléments électroluminescents le long de chemins qui ne sont pas interrompus par lesdites griffes de prise (127).

6. Injecteur de liquide (100) pour injecter un liquide depuis une seringue de liquide (200) ayant un cylindre (201) avec une bride de cylindre (203) disposée sur et autour d'une extrémité de celui-ci et un piston (202) inséré on coulissement dans ledit cylindre (201) et ayant une bride de piston (204) disposée sur et autour d'une extrémité de celui-ci, ledit injecteur de liquide (100) étant disposé de façon à détenir et à déplacer au moins ladite bride de cylindre (203) et ladite bride de piston (204) l'une par rapport à l'autre, ledit injecteur de liquide (100) comprenant :
un mécanisme de saisie de cylindre (110) pour saisir ledit cylindre (201), de sorte que ledit cylindre (201) ait une direction longitudinale orientée vers l'avant et vers l'arrière ;
un système de poussée de piston (126) supporté en coulissement dans des directions vers l'avant et vers l'arrière pour pousser ledit piston (202) au moins vers l'avant;
une paire de griffes de prise (127) montées de manière à pouvoir s'ouvrir et se fermer latéralement sur ledit système de poussée de piston (126), afin de mettre en prise individuellement les bords gauche et droit d'une face avant de ladite bride de piston (204); et
des moyens de détection de prise pour détecter quand ladite bride de piston (204) est saisie par lesdites griffes de prise (127),
**caractérisé en ce que** lesdits moyens de détection de prise comprennent des moyens pour détecter quand lesdites griffes de prise (127) sont ouvertes à partir d'une position fermée initiale dans des côtés opposés, puis fermées.
